# EUROPEAN PATENT APPLICATION

(11) **EP 4 674 454 A1**
(43) Date of publication of application: **07.01.2026**
(21) Application number: 24778070.3
(22) Date of filing: 27.03.2024
(51) Int. Cl.: A61M 11/04, A61M 11/00, A61M 15/00, A61M 15/06, A61M 16/10, A61K 9/72, A61K 31/55, A24B 15/167

(54) **LIQUID PREPARATION FOR ATOMIZATION IN ELECTRONIC ATOMIZER, CARTRIDGE AND AEROSOL GENERATION SYSTEM**

(30) Priority: 29.03.2023 CN 202310351111
(71) Applicant: Shenzhen First Union Technology Co., Ltd., Shenzhen, Guangdong 518000 (CN)
(72) Inventor: LU, Jin, Shenzhen, Guangdong 518000 (CN); ZHAO, Jing, Shenzhen, Guangdong 518000 (CN); LIU, Zhang, Shenzhen, Guangdong 518000 (CN); DENG, Jingjing, Shenzhen, Guangdong 518000 (CN); CHU, Ming, Shenzhen, Guangdong 518000 (CN); HUANG, Fuyuan, Shenzhen, Guangdong 518000 (CN); XU, Zhongli, Shenzhen, Guangdong 518000 (CN); LI, Yonghai, Shenzhen, Guangdong 518000 (CN)
(74) Representative: Ran, Handong
(86) International application number: PCT/CN2024/084103
(87) International publication number: WO 2024/199284

(57) **Abstract**

The present application discloses a liquid preparation for atomization in an electronic atomizer, a cartridge and an aerosol generation system. The liquid preparation comprises a solvent and azelastine hydrochloride.

## Description

This application claims priority to Chinese Patent Application No. 202310351111.6, entitled "LIQUID PREPARATION FOR ATOMIZATION IN ELECTRONIC ATOMIZER, CARTRIDGE AND AEROSOL GENERATION SYSTEM" and filed on March 29, 2023, which is incorporated herein by reference in its entirety.

### TECHNICAL FIELD

Embodiments of the present application relate to the field of aerosol-generating devices, and more particularly to a liquid preparation for atomization in an electronic atomizer, a cartridge and an aerosol generation system.

### BACKGROUND

Azelastine Hydrochloride (AH) is a potential long-acting antiallergic compound having the characteristics of H1 receptor antagonists. It has anti-allergic, antiasthmatic and antihistamine properties, and is usually used clinically in the treatment of anti-allergic rhinitis and asthma.

Azelastine hydrochloride used as a medicine is prepared into tablets or granules for oral administration by patients, prepared as a nasal spray formulation for intranasal delivery, or prepared as eye drops for ophthalmic use.

Azelastine hydrochloride used as tablets and granules has a strong bitter taste. Azelastine hydrochloride used as a nasal spray formulation and eye drops is inconvenient for users to carry and use promptly for symptom relief.

### SUMMARY

An embodiment of the present application provides a liquid preparation for atomization in an electronic atomizer, including:
a solvent; and
azelastine hydrochloride.

In the above embodiment, azelastine hydrochloride is formulated into a liquid preparation for atomization in an electronic atomizer, and the liquid preparation is fed to an electronic atomizer for atomization to generate an aerosol for a user to inhale. Existing electronic atomizers that can atomize azelastine hydrochloride for inhalation are configured to be portable, which is convenient for users to carry, increases patient compliance, and broadens the scope of clinical therapeutic applications. In addition, the boiling point of azelastine hydrochloride is about 534°C, and the working temperature range of existing electronic atomizers is about 100°C to 500°C. Therefore, azelastine hydrochloride can form an azeotrope with a commonly used solvent in the electronic atomizer, thereby generating an aerosol. Further, azelastine hydrochloride is prepared into a liquid preparation and an atomization test is performed on the liquid preparation using an electronic atomizer. It is found through the atomization test that the atomization conversion rate of azelastine hydrochloride is high. Therefore, in the process of a user inhaling the aerosol generated by the atomization of the liquid preparation, azelastine hydrochloride is inhaled into the lungs and reaches the alveoli, and can be quickly absorbed and enter into the blood circulation, achieving significantly higher biological effectiveness than that of oral administration. Therefore, with the formulation of azelastine hydrochloride into a liquid preparation, the efficacy of azelastine hydrochloride can be better exerted by atomization of the liquid preparation for inhalation.

The azelastine hydrochloride is a pharmaceutical grade raw material and can be prepared by various methods reported in the prior art.

In some embodiments, the mass percentage of the azelastine hydrochloride in the liquid preparation is 0.01%-3%; or the mass percentage of the azelastine hydrochloride in the liquid preparation is 0.1%-1%.

When azelastine hydrochloride is used as tablets, the daily dosage limit of azelastine hydrochloride is no more than 4 mg for an adult. Therefore, the mass percentage content of azelastine hydrochloride is controlled within 3% considering the amount of azelastine hydrochloride inhaled from the electronic atomizer by each vaping and the number of times a user vapes per day, allowing the user to use the electronic atomizer multiple puffing in one day to relieve the symptoms of rhinitis or asthma.

Further, it is found by testing the atomization effect of the liquid preparation containing azelastine hydrochloride that the mass percentage of azelastine hydrochloride in the liquid preparation is controlled at about 0.1% to 1%, and the conversion rate of azelastine hydrochloride in the liquid preparation into an aerosol is substantially 90% or more, even close to 100%. Therefore, controlling the mass percentage of azelastine hydrochloride in the liquid preparation within 0.1% to 1% is beneficial to improving the conversion rate.

In some embodiments, the solvent includes at least one of propylene glycol, vegetable glycerin, water, or ethanol.

In some embodiments, the mass percentage of the propylene glycol in the liquid preparation is 20%-80%.

In some embodiments, the mass percentage of the vegetable glycerin in the liquid preparation is 30%-70%.

The propylene glycol may be 1,2-propanediol or 1,3-propanediol.

As commonly used solvents in electronic atomizers propylene glycol and vegetable glycerin have excellent performance in increasing the amount of aerosol generated and controlling the fluidity of liquid preparations, so propylene glycol and vegetable glycerin are used as main solvent components. In addition, an appropriate amount of water or ethanol may be added as a solvent according to an increase in the content of azelastine hydrochloride by mass percentage in the liquid preparation, thereby increasing the solubility of azelastine hydrochloride in the liquid preparation.

In some embodiments, the atomization conversion rate of the azelastine hydrochloride is not less than 90%; or the atomization conversion rate of the azelastine hydrochloride is not less than 80%; or the atomization conversion rate of the azelastine hydrochloride is not less than 70%; or the atomization conversion rate of the azelastine hydrochloride is not less than 60%.

In some embodiments, the particle size of an aerosol generated by the electronic atomizer by atomizing the azelastine hydrochloride in the liquid preparation for atomization in the electronic atomizer ranges from 0.2 µm to 3 µm.

The particle sizes of most particles of the aerosol of azelastine hydrochloride is concentrated in the range of 0.2 µm to 1.5 µm.

By adopting the atomization test method provided in the embodiments of the present application, it is found that the atomization conversion rate of azelastine hydrochloride is not less than 90% when the content of azelastine hydrochloride in the liquid preparation is approximately set to be within a range of 0.1% to 1% (mass percentage). When the content of azelastine hydrochloride in the liquid preparation is adjusted to a certain range, the conversion rate of azelastine hydrochloride is not less than 80%. When the content of azelastine hydrochloride in the liquid preparation is further adjusted to another range, or a different electronic atomizer is used, the conversion rate of azelastine hydrochloride is not less than 70%, or the conversion rate of azelastine hydrochloride is not less than 60%. Different electronic atomizers are configured to have different heating powers or use different atomizing core assemblies.

In some embodiments, the liquid preparation further includes at least one of an edible essence, a sweetener, or a cooling agent.

The edible essence is used to increase the taste and aromatic flavor of the aerosol generated by atomization of the liquid preparation. The type of the edible essence includes one or more of fruit flavor, floral flavor, mint flavor, or tea flavor.

The mass percentage of the edible essence in the liquid preparation is approximately in a range of 0 to 10%, and the specific content of the edible essence in the liquid preparation may be set to any content value in the range of 0 to 10% according to a requirement for adjusting the taste of the liquid preparation. For example, the mass percentage of the edible essence is 5%.

The sweetener is used to increase the sweetness of the aerosol generated by atomization of the liquid preparation. It should be noted that azelastine hydrochloride has a certain bitter taste, and thus adding an appropriate amount of sweetener to the liquid preparation helps to improve the taste of the liquid preparation, shields the bitter taste of azelastine hydrochloride, and thus greatly improves the pleasure of vaping of the user. The type of the sweetener may be selected from one or more of cyclamate, xylitol, mogroside, anselfame, saccharin sodium, sucralose, aspartame, alitame, neohesperidin dihydrochalcone, neotame, and steviol glycoside.

The mass percentage of the sweetener in the liquid preparation is approximately in a range of 0% to 10%, and the specific content of the sweetener in the liquid preparation may be set to any content value in the range of 0% to 10% according to a requirement for adjusting the taste of the liquid preparation. For example, the mass percentage of the sweetener in the liquid preparation is 1%.

The cooling agent is used to increase the cooling effect of the aerosol generated by atomization of the liquid preparation, thereby enhancing the pleasure of the user in the process of inhaling the aerosol. Cooling agents suitable for use in the liquid preparation may be one or more of menthol, menthone, isomenthone, menthols, menthyl ethers, menthyl esters, WS-23 (2-isopropyl-N,2,3-trimethylbutyramide), WS-3 (N-ethyl-p-menthyl-3-carboxamide), WS-5 (N-(ethoxycarbonylmethyl)-p-alkane-3-carboxamide), or WS-12 (N-(4-methoxyphenyl)-p-menthyl-3-carboxamide).

The mass percentage of the cooling agent in the liquid preparation is approximately in a range of 0 to 10%, and the specific content of the cooling agent in the liquid preparation may be set to any content value in the range of 0 to 1% according to a requirement for adjusting the taste of the liquid preparation. For example, the mass percentage of the cooling agent in the liquid preparation is 2%.

One or more edible essences, one or more sweeteners, and one or more cooling agents are added into the liquid preparation containing azelastine hydrochloride and uniformly mixed. Mixing methods include stirring at normal temperature, stirring while heating, ultrasonic mixing, stirring with a stirring paddle, magnetic stirring, or other mixing methods with stirring in the prior art.

An embodiment of the present application also provides a cartridge for use in an electronic atomizer. The cartridge stores the liquid preparation.

The cartridge may be configured as an independently sold closed container and assembled to an electronic atomizer, and the liquid preparation in the cartridge can flow to an atomizing core assembly on the electronic atomizer and be atomized by the atomizing core assembly to generate an aerosol.

The cartridge may also be configured as a non-detachable component of an electronic atomizer, and the liquid preparation in the cartridge can flow to an atomizing core assembly on the electronic atomizer and be atomized by the atomizing core assembly to generate an aerosol. When the liquid preparation inside the cartridge is used up, at least a portion of the electronic atomizer may be disposed of.

An embodiment of the present application also provide an aerosol generation system including the liquid preparation and an electronic atomizer configured to atomize the liquid preparation to generate an aerosol.

In some embodiments, the electronic atomizer includes a ceramic wick atomizer or a cotton wick atomizer.

### BRIEF DESCRIPTION OF THE DRAWINGS

To describe the technical solutions of the embodiments of the present application more clearly, the following briefly introduces the accompanying drawings required for describing the embodiments of the present application. Apparently, the drawings depicted below are merely embodiments of the present application, and those skilled in the art can obtain other drawings based on these drawings without any creative efforts.
FIG. 1 is a high-performance liquid chromatography (HPLC) chromatogram of a liquid preparation containing azelastine hydrochloride according to some embodiments of the present application;
FIG. 2 is an HPLC chromatogram of an aerosol generated by atomization of a liquid preparation containing azelastine hydrochloride according to some embodiments of the present application; and
FIG. 3 is a diagram showing the aerosol particle size distribution of azelastine hydrochloride according to some embodiments of the present application.

### DETAILED DESCRIPTION

For ease of understanding of the present application, the present application will be described in more detail below with reference to specific embodiments. The examples are merely used for providing a clearer understanding of the technical features, objectives, and effects of the present application, and are not intended to limit the present application.

The methods given in examples below are all conventional methods, unless otherwise stated.

The materials and instruments used in examples below are all commercially available, unless otherwise stated.

In Example 1 of the present application, azelastine hydrochloride was formulated as an inhalable liquid preparation, and the stability of azelastine hydrochloride before and after atomization is analyzed using an HPLC system.

The materials and instruments used in Example 1 are listed below.

Materials: Azelastine hydrochloride (AH), 1,2-propanediol, vegetable glycerin, triethylamine, phosphoric acid, methanol, and acetonitrile.

Instruments: LC-20A HPLC system from Shimadzu, Japan, Labsolutions workstation, Cerulean smoking machine, and Sartorius BSA224S electronic balance.
1. A liquid preparation containing azelastine hydrochloride was formulated.

The mass percentages of components in the liquid preparation are as follows: azelastine hydrochloride: 0.5%; vegetable glycerin: 50%; water: 29.5%; and 1,2-propanediol: 20%.

2. An aerosol generated by atomization of azelastine hydrochloride was captured.

An appropriate amount of the liquid preparation was fed to an electronic atomizer. The electronic atomizer was vaped by a Cerulean smoking machine. A single Cambridge filter was used to trap the aerosol. Parameters used by the smoking machine included: a vaping capacity of 55 mL, a vaping frequency of 30s, a vaping duration of 3s, and 50 times of vaping. The aerosol captured on the Cambridge filter was collected, and shaken and extracted using the initial mobile phase.

### 3. Chromatographic conditions

A Thermo Fisher Scientific Acclaim 120 C18 (4.6*250 mm, 5 µm) column was used, with reference to the 2015 edition of the Chinese Pharmacopoeia for the determination of AH-related substances. Mobile phase: 4% triethylamine solution (pH adjusted to 6.0 with phosphoric acid)-acetonitrile-methanol (50:18:32). Flow rate: 1.0 mL/min. Detection wavelength: 289 nm. Column temperature: 30°C.

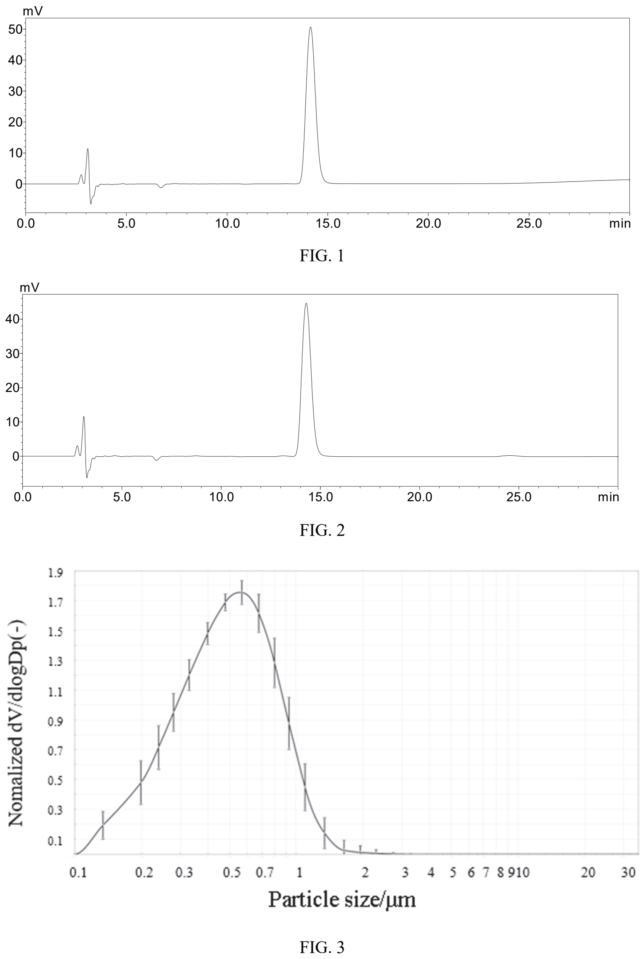

FIG. 1 is a chromatogram of the liquid preparation containing azelastine hydrochloride. FIG. 2 is a chromatogram of the aerosol generated by atomization of azelastine hydrochloride. It can be seen from FIG. 1 and FIG. 2 that the peak pattern of the chromatographic peak of azelastine hydrochloride had no significant change after the atomization of azelastine hydrochloride, and there was substantially no extra peak in FIG. 2 compared with FIG. 1, indicating that the aerosol obtained by the atomization of azelastine hydrochloride had a stable chemical composition, and the chemical structure of the azelastine hydrochloride will not be cleaved due to the electric heating atomization process, so the efficacy of azelastine hydrochloride is not affected.

In Example 2 of the present application, a liquid preparation in which the mass percentage of azelastine hydrochloride was 0.5% was used as an example to calculate the conversion rate of generating an aerosol from atomization of azelastine hydrochloride.

The materials and instruments used in Example 2 were as follows.

Materials: Azelastine hydrochloride (AH), 1,2-propanediol, vegetable glycerin, triethylamine, phosphoric acid, methanol, and acetonitrile.

Instruments: LC-20A HPLC system from Shimadzu, Japan, Labsolutions workstation, Cerulean smoking machine, and Sartorius BSA224S electronic balance.
1. A liquid preparation containing azelastine hydrochloride was formulated.

The mass percentages of components in the liquid preparation are as follows: azelastine hydrochloride: 0.5%; vegetable glycerin: 50%; water: 29.5%; and 1,2-propanediol: 20%.

2. An aerosol generated by atomization of azelastine hydrochloride was captured.

An appropriate amount of the liquid preparation was fed to an electronic atomizer. The electronic atomizer was vaped by a Cerulean smoking machine. A single Cambridge filter was used to trap the aerosol. Parameters used by the smoking machine included: a vaping capacity of 55 mL, a vaping frequency of 30s, a vaping duration of 3s, and 50 times of vaping. The aerosol captured on the Cambridge filter was collected, and shaken and extracted using the initial mobile phase.

### 3. Chromatographic conditions used by the HPLC system

A Thermo Fisher Scientific Acclaim 120 C18 (4.6*250 mm, 5 µm) column was used, with reference to the 2015 edition of the Chinese Pharmacopoeia for the determination of AH-related substances. Mobile phase: 4% triethylamine solution (pH adjusted to 6.0 with phosphoric acid)-acetonitrile-methanol (50:18:32). Flow rate: 1.0 mL/min. Detection wavelength: 289 nm. Column temperature: 30°C.

4. The conversion rate of generating an aerosol from azelastine hydrochloride through atomization was tested.

4.1 A blank control solution and a liquid preparation containing azelastine hydrochloride were formulated, and an azelastine hydrochloride aerosol was captured.

Blank control: initial mobile phase.

Azelastine hydrochloride liquid preparation: An appropriate amount of the liquid preparation was taken and diluted with the initial mobile phase.

Extraction of azelastine hydrochloride aerosol: A Cambridge filter used to collect the aerosol was taken, and shaken and extracted using the initial mobile phase.

### 4.2 Calculation method

The atomization conversion rate was calculated based on the following formula: A= w1/w2*100%
where A= atomization conversion rate
w1 = mass percentage of azelastine hydrochloride in the aerosol
w2 = mass percentage of azelastine hydrochloride in the liquid preparation

The test results of the atomization conversion rate of the liquid preparation in which the mass percentage of azelastine hydrochloride was 0.5% are as shown in Table 1 below.

**Table 1 Atomization test results of 0.5% azelastine hydrochloride**

| Sample | Aerosol mass /g | Sample concentration/g·mL⁻¹ | w/% | A/% |
|---|---|---|---|---|
| Aerosol | 0.264 | 0.042 | 0.485 | 97.98 |
| Liquid preparation | - | 0.046 | 0.495 | |

| | | | | |
|---|---|---|---|---|
| Note: w: the mass percentage of azelastine hydrochloride in the aerosol/liquid preparation | | | | |

### A: atomization conversion rate

FIG. 1 is a chromatogram of a liquid preparation in which the mass percentage of azelastine hydrochloride is 0.5%. FIG. 2 is a chromatogram of an aerosol generated by atomization of the liquid preparation containing azelastine hydrochloride. As measured by the above calculation method, the atomization conversion rate of azelastine hydrochloride whose mass percentage was 0.5% was 97.98, so the chemical composition of azelastine hydrochloride was stable when converted into an aerosol, and the conversion efficiency was high.

In Example 3 of the present application, a liquid preparation in which the mass percentage of azelastine hydrochloride was 1% was used as an example to calculate the conversion rate of generating an aerosol from atomization of azelastine hydrochloride.

The materials and instruments used in Example 3 were as follows.

Materials: Azelastine hydrochloride (AH), 1,2-propanediol, vegetable glycerin, triethylamine, phosphoric acid, methanol, and acetonitrile.

Instruments: LC-20A HPLC system from Shimadzu, Japan, Labsolutions workstation, Cerulean smoking machine, and Sartorius BSA224S electronic balance.
1. A liquid preparation containing azelastine hydrochloride was formulated.

The mass percentages of components in the liquid preparation are as follows: azelastine hydrochloride: 1%; vegetable glycerin: 50%; water: 29%; and 1,2-propanediol: 20%.

2. An aerosol generated by atomization of azelastine hydrochloride was captured.

An appropriate amount of the liquid preparation was fed to an electronic atomizer. The electronic atomizer was vaped by a Cerulean smoking machine. A single Cambridge filter was used to trap the aerosol. Parameters used by the smoking machine included: a vaping capacity of 55 mL, a vaping frequency of 30s, a vaping duration of 3s, and 50 times of vaping. The aerosol captured on the Cambridge filter was collected, and shaken and extracted using the initial mobile phase.

### 3. Chromatographic conditions used by the HPLC system

A Thermo Fisher Scientific Acclaim 120 C18 (4.6*250 mm, 5 µm) column was used, with reference to the 2015 edition of the Chinese Pharmacopoeia for the determination of AH-related substances. Mobile phase: 4% triethylamine solution (pH adjusted to 6.0 with phosphoric acid)-acetonitrile-methanol (50:18:32). Flow rate: 1.0 mL/min. Detection wavelength: 289 nm. Column temperature: 30°C.

4. The conversion rate of generating an aerosol from azelastine hydrochloride through atomization was tested.

4.1 A blank control solution and a liquid preparation containing azelastine hydrochloride were formulated, and an azelastine hydrochloride aerosol was captured.

Blank control: initial mobile phase.

Liquid preparation containing azelastine hydrochloride: An appropriate amount of the liquid preparation was taken and diluted with the initial mobile phase.

Extraction of azelastine hydrochloride aerosol: A Cambridge filter used to collect the aerosol was taken, and shaken and extracted using the initial mobile phase.

### 4.2 Calculation method

The atomization conversion rate was calculated based on the following formula: A= w1/w2*100%
where A= atomization conversion rate
w1 = mass percentage of azelastine hydrochloride in the aerosol
w2 = mass percentage of azelastine hydrochloride in the liquid preparation

The test results of the atomization conversion rate of the liquid preparation in which the mass percentage of azelastine hydrochloride was 1% are as shown in Table 2 below.

**Table 2 Atomization test results of 1% azelastine hydrochloride**

| Sample | Aerosol mass /g | Sample concentration/g·mL⁻¹ | w/% | A/% |
|---|---|---|---|---|
| Aerosol | 0.249 | 0.04 | 0.986 | 93.54 |
| Liquid preparation | - | 0.038 | 1.054 | |

| | | | | |
|---|---|---|---|---|
| Note: w: the mass percentage of azelastine hydrochloride in the aerosol/liquid preparation | | | | |

### A: atomization conversion rate

As measured by the above calculation method, the atomization conversion rate of azelastine hydrochloride whose mass percentage was 1% was 93.54. It can be seen that the atomization conversion rate of azelastine hydrochloride in the liquid preparation in which the mass percentage content of azelastine hydrochloride was 1% was still greater than 90%, indicating that the aerosol generated by the electric heating atomization of azelastine hydrochloride had a stable chemical composition.

In Example 4 of the present application, a liquid preparation in which the mass percentage of azelastine hydrochloride was 0.5% was used as an example, multiple times of vaping were performed, the weight of azelastine hydrochloride in each vaping of the aerosol was analyzed to analyze the uniformity of conversion of azelastine hydrochloride into the aerosol.

The mass percentages of components in the liquid preparation were as follows: azelastine hydrochloride: 0.5%; vegetable glycerin: 50%; 1,2-propanediol: 20%; and water: 29.5%.

The liquid preparation of the above example was added to an electronic atomizer. The electronic atomizer was vaped by a Cerulean smoking machine. A single Cambridge filter was used to trap the aerosol. The Cambridge filter was collected once every 25 times of vaping. The electronic atomizer was vaped for a total of 250 times. The measured aerosol conversion rate results are as shown in Table 3 below.

**Table 3 Test results of atomization conversion rate of azelastine hydrochloride**

| Serial number | Number of times of vaping | Aerosol weight /g | A/% |
|---|---|---|---|
| 1 | 25 | 0.1241 | 98.52 |
| 2 | 50 | 0.1259 | 98.98 |
| 3 | 75 | 0.1197 | 94.26 |
| 4 | 100 | 0.1170 | 93.62 |
| 5 | 125 | 0.1131 | 93.15 |
| 6 | 150 | 0.1131 | 95.67 |
| 7 | 175 | 0.1156 | 96.64 |
| 8 | 200 | 0.1159 | 97.95 |
| 9 | 225 | 0.1183 | 95.72 |
| 10 | 250 | 0.1191 | 96.21 |

| | | | |
|---|---|---|---|
| Note: A: atomization conversion rate | | | |

It can be seen from Table 3 that the difference in aerosol weight collected per 25 times of vaping was small, and the conversion rates were greater than 90%. It can be seen that the conversion rate of the liquid preparation containing azelastine hydrochloride was relatively stable, indicating good uniformity.

It should be noted that the number of times of vaping for most of existing electronic atomizers is about 500. The above experiment tested the quality stability of azelastine hydrochloride in every 25 times of vaping of the aerosol, and it is found that the uniformity was good. Therefore, it is fully proved that symptoms of a user can be uniformly relieved as the user repeatedly inhales the aerosol containing azelastine hydrochloride, and azelastine hydrochloride is suitable for atomization by an electronic atomizer for inhalation.

In Example 5 of the present application, the particle size distribution of the azelastine hydrochloride aerosol was measured using a HELOS BR laser particle size analyzer from Sympatec, Germany, and a smoking machine made by Shenzhen FirstUnion Technology Co., Ltd. was equipped.

The test method was as follows.

An R1 lens (with a test range of 0.1 µm to 35 µm) was selected, particle size segments with equal logarithmic spacing were collected respectively, and there were 10 channels of actual measured signals in the sub-micron range, with high particle size resolution. Electronic cigarette vaping mode: a vaping capacity of 55 mL, a vaping duration of 3s, a vaping interval of 30s, and a square wave vaping curve. Each sample was tested in parallel for three times. Three times of vaping were performed for each time of testing. The test results were averaged. One time slice was obtained every 100 milliseconds, and a total of 30 slices were obtained within 3 seconds of vaping. Flow rate: 18.3 mL/min.

**Table 4 Results of particle size test of azelastine hydrochloride**

| Sample | Dv10/µm | Dv50/µm | Dv90/µm | Span |
|---|---|---|---|---|
| Azelastine hydrochloride | 0.22 | 0.51 | 0.95 | 1.45 |

The results in Table 4 show that the measured median particle size of the azelastine hydrochloride aerosol was 0.51 µm. The particle size distribution of the azelastine hydrochloride aerosol is as shown in FIG. 3, in which the aerosol exhibits a single peak approximate lognormal distribution, the aerosol particle size is mainly distributed in the range of 0.2 µm to 1.5 µm, and a few aerosol particles have a particle size of 1.5 µm to 3 µm.

The aerosol particle size of azelastine hydrochloride is small, so the aerosol has a more delicate taste.

Example 5 of the present application provides a liquid preparation containing azelastine hydrochloride, which is suitable for atomization by an electronic atomizer. The mass percentage of azelastine hydrochloride in the liquid preparation ranges from 0.01% to 3%. Alternatively, the mass percentage of azelastine hydrochloride in the liquid preparation ranges from 0.1% to 1%.

When azelastine hydrochloride is used as tablets, the daily dosage limit of azelastine hydrochloride is no more than 4 mg for an adult. Therefore, the content of azelastine hydrochloride is controlled within 3% considering the amount of azelastine hydrochloride inhaled from the electronic cigarette by each vaping and the number of times a user vapes per day, allowing the user to use the electronic atomizer multiple puffing in one day to relieve the symptoms of rhinitis or asthma.

Further, it is found by testing the atomization effect of the liquid preparation containing azelastine hydrochloride that the mass percentage of azelastine hydrochloride in the liquid preparation is controlled at about 0.1% to 1%, and the conversion rate of azelastine hydrochloride in the liquid preparation into an aerosol is substantially 90% or more, even close to 100%. Therefore, controlling the mass percentage of azelastine hydrochloride in the liquid preparation within 0.1% to 1% is beneficial to improving the conversion rate.

In some embodiments, the solvent includes at least one of propylene glycol, vegetable glycerin, water, or ethanol.

In some embodiments, the mass percentage of propylene glycol in the inhalable preparation composition ranges from 20% to 80%.

In some embodiments, the mass percentage of vegetable glycerin in the inhalable preparation composition ranges from 30% to 70%.

The propylene glycol may be 1,2-propanediol or 1,3-propanediol.

As commonly used solvents in electronic atomizers propylene glycol and vegetable glycerin have excellent performance in increasing the amount of aerosol generated and controlling the fluidity of liquid preparations, so propylene glycol and vegetable glycerin are used as main solvent components. In addition, an appropriate amount of water or ethanol may be added as a solvent according to an increase in the content of azelastine hydrochloride by mass percentage in the liquid preparation, thereby increasing the solubility of azelastine hydrochloride in the liquid preparation.

In some embodiments, the atomization conversion rate of the azelastine hydrochloride is not less than 90%; or the atomization conversion rate of the azelastine hydrochloride is not less than 80%; or the atomization conversion rate of the azelastine hydrochloride is not less than 70%; or the atomization conversion rate of the azelastine hydrochloride is not less than 60%.

By adopting the atomization test method provided in the embodiments of the present application, it is found that the atomization conversion rate of azelastine hydrochloride is not less than 90% when the content of azelastine hydrochloride in the liquid preparation is approximately set to be within a range of 0.1% to 1% (mass percentage). When the content of azelastine hydrochloride in the liquid preparation is adjusted to a certain range, the conversion rate of azelastine hydrochloride is not less than 80%. When the content of azelastine hydrochloride in the liquid preparation is further adjusted to another range, or a different electronic atomizer is used, the conversion rate of azelastine hydrochloride is not less than 70%, or the conversion rate of azelastine hydrochloride is not less than 60%. Different electronic atomizers are configured to have different heating powers or use different atomizing core assemblies.

In some embodiments, the liquid preparation further includes at least one of an edible essence, a sweetener, or a cooling agent.

The edible essence is used to increase the taste and aromatic flavor of the aerosol generated by atomization of the liquid preparation. The type of the edible essence includes one or more of fruit flavor, floral flavor, mint flavor, or tea flavor.

The mass percentage of the edible essence in the liquid preparation is approximately in a range of 0 to 10%, and the specific content of the edible essence in the liquid preparation may be set to any content value in the range of 0 to 10% according to a requirement for adjusting the taste of the liquid preparation. For example, the mass percentage of the edible essence is 5%.

The sweetener is used to increase the sweetness of the aerosol generated by atomization of the liquid preparation. It should be noted that azelastine hydrochloride has a certain bitter taste, and thus adding an appropriate amount of sweetener to the liquid preparation helps to improve the taste of the liquid preparation, shields the bitter taste of azelastine hydrochloride, and thus greatly improves the pleasure of vaping of the user. The type of the sweetener may be selected from one or more of cyclamate, xylitol, mogroside, anselfame, saccharin sodium, sucralose, aspartame, alitame, neohesperidin dihydrochalcone, neotame, and steviol glycoside.

The mass percentage of the sweetener in the liquid preparation is approximately in a range of 0% to 10%, and the specific content of the sweetener in the liquid preparation may be set to any content value in the range of 0% to 10% according to a requirement for adjusting the taste of the liquid preparation. For example, the mass percentage of the sweetener in the liquid preparation is 1%.

The cooling agent is used to increase the cooling effect of the aerosol generated by atomization of the liquid preparation, thereby enhancing the pleasure of the user in the process of inhaling the aerosol. Cooling agents suitable for use in the liquid preparation may be one or more of menthol, menthone, isomenthone, menthols, menthyl ethers, menthyl esters, WS-23 (2-isopropyl-N,2,3-trimethylbutyramide), WS-3 (N-ethyl-p-menthyl-3-carboxamide), WS-5 (N-(ethoxycarbonylmethyl)-p-alkane-3-carboxamide), or WS-12 (N-(4-methoxyphenyl)-p-menthyl-3-carboxamide).

The mass percentage of the cooling agent in the liquid preparation is approximately in a range of 0 to 10%, and the specific content of the cooling agent in the liquid preparation may be set to any content value in the range of 0 to 1% according to a requirement for adjusting the taste of the liquid preparation. For example, the mass percentage of the cooling agent in the liquid preparation is 2%.

One or more edible essences, one or more sweeteners, and one or more cooling agents are added into the liquid preparation containing azelastine hydrochloride and uniformly mixed. Mixing methods include stirring at normal temperature, stirring while heating, ultrasonic mixing, stirring with a stirring paddle, magnetic stirring, or other mixing methods with stirring in the prior art.

It should be noted that the inventors have tested the atomization performance of more than 20 pharmaceutical reagents, and found that most pharmaceutical reagents are not suitable for atomization by an electronic atomizer for inhalation. For example, the atomization conversion rate of clenbuterol hydrochloride is less than 10%, the atomization conversion rate of cromolyn sodium is less than 15%, and the atomization conversion rate of terbutaline sulfate is less than 50%. For such pharmaceutical reagents with a low conversion rate, the content of the therapeutic effective component in the aerosol is low, and even if a user inhales the aerosol multiple times, symptoms of the user cannot be relieved. For the liquid preparation containing azelastine hydrochloride in the embodiments of the present application, the conversion rate of converting the therapeutic effective component in the liquid preparation into an aerosol through atomization by an electronic atomizer is high with good uniformity, and an unexpected technical effect is produced. Therefore, azelastine hydrochloride is suitable to be formulated into a liquid preparation for atomization by an electronic atomizer to generate an aerosol for a user to inhale to relieve symptoms.

Example 6 of the present application provides a cartridge for use in an electronic atomizer. A liquid preparation containing azelastine hydrochloride is stored in the cartridge.

The cartridge may be configured as an independently sold closed container and assembled to an electronic atomizer, and the liquid preparation in the cartridge can flow to an atomizing core assembly on the electronic atomizer and be atomized by the atomizing core assembly to generate an aerosol.

The cartridge may also be configured as a non-detachable component of an electronic atomizer, and the liquid preparation in the cartridge can flow to an atomizing core assembly on the electronic atomizer and be atomized by the atomizing core assembly to generate an aerosol. When the liquid preparation inside the cartridge is used up, at least a portion of the electronic atomizer may be disposed of.

Example 7 of the present application provides an aerosol generation system including the liquid preparation and an electronic atomizer configured to atomize the liquid preparation to generate an aerosol.

In some embodiments, the electronic atomizer includes a ceramic wick atomizer or a cotton wick atomizer.

The ceramic wick atomizer includes a porous wicking structure and a heating element bonded to the porous wicking structure. The porous wicking structure is made of a porous material, which includes microporous ceramics, microporous glass ceramics, microporous glass, foamed metal, etc. The heating element may be made of a material having an appropriate impedance, which includes a metal material, a metal alloy, graphite, carbon, an electrically conductive ceramic, or a composite material of a ceramic material and a metal material. Suitable metal or alloy materials include at least one of nickel, cobalt, zirconium, titanium, a nickel alloy, a cobalt alloy, a zirconium alloy, a titanium alloy, a nickel-chromium alloy, a nickel-iron alloy, an iron-chromium alloy, a titanium alloy, an iron-manganese-aluminum-based alloy, stainless steel, etc.

The cotton wick atomizer includes a wicking element and a heating element bonded to the wicking element. The wicking element is made of fiber cotton. The material of the heating element in the cotton wick atomizer is approximately the same as the material of the heating element in the ceramic wick atomizer.

In addition to the atomizing core assembly, the electronic atomizer includes a battery assembly configured to supply power.

The electronic atomizer is preferably configured as a small portable device, which is convenient for a user to carry and vape at any time, to relieve the symptoms of rhinitis or asthma.

Finally, it should be noted that, the foregoing embodiments are merely used for describing the technical solutions of the present application, but are not intended to limit the present application. Based on the idea of the present application, the technical features in the above embodiments or in different embodiments may be combined, the steps may be implemented in any order, and there may be many other variations of the different aspects of the present application described above, which are not described in detail for the sake of conciseness. It should be understood by a person of ordinary skill in the art that although the present application has been described in detail with reference to the foregoing embodiments, modifications can be made to the technical solutions described in the foregoing embodiments, or equivalent replacements can be made to some technical features in the technical solutions, as long as such modifications or replacements do not cause the essence of corresponding technical solutions to depart from the scope of the technical solutions of the embodiments of the present application.

## Claims

1. A liquid preparation for atomization in an electronic atomizer, comprising:
a solvent; and
azelastine hydrochloride.

2. The liquid preparation for atomization in an electronic atomizer according to claim 1, wherein mass percentage of the azelastine hydrochloride in the liquid preparation is 0.01%-3%; or the mass percentage of the azelastine hydrochloride in the liquid preparation is 0.1%-1%.

3. The liquid preparation for atomization in an electronic atomizer according to claim 1, wherein the solvent comprises at least one of propylene glycol, vegetable glycerin, water, or ethanol.

4. The liquid preparation for atomization in an electronic atomizer according to claim 3, wherein mass percentage of the propylene glycol in the liquid preparation is 20%-80%.

5. The liquid preparation for atomization in an electronic atomizer according to claim 3, wherein mass percentage of the vegetable glycerin in the liquid preparation is 30%-70%.

6. The liquid preparation for atomization in an electronic atomizer according to claim 1, wherein an atomization conversion rate of the azelastine hydrochloride is not less than 90%; or
the atomization conversion rate of the azelastine hydrochloride is not less than 80%; or
the atomization conversion rate of the azelastine hydrochloride is not less than 70%; or
the atomization conversion rate of the azelastine hydrochloride is not less than 60%.

7. The liquid preparation for atomization in an electronic atomizer according to claim 1, wherein a particle size of an aerosol generated by the electronic atomizer by atomizing the azelastine hydrochloride in the liquid preparation for atomization in the electronic atomizer ranges from 0.2 µm to 3 µm.

8. The liquid preparation for atomization in an electronic atomizer according to claim 1, wherein the liquid preparation further comprises at least one of an edible essence, a sweetener, or a cooling agent.

9. A cartridge, applied to an electronic atomizer, wherein the liquid preparation for atomization in an electronic atomizer according to any one of claims 1-8 is stored in the cartridge.

10. An aerosol generation system, comprising the liquid preparation for atomization in an electronic atomizer according to any one of claims 1-8 and an electronic atomizer configured to atomize the liquid preparation to generate an aerosol.

11. The aerosol generation system according to claim 10, wherein the electronic atomizer comprises a ceramic wick atomizer or a cotton wick atomizer.
